# EUROPEAN PATENT SPECIFICATION

(11) **EP 1 207 885 B1**
(45) Date of publication and mention of the grant of the patent: **28.09.2005**
(21) Application number: 01925860.7
(22) Date of filing: 30.03.2001
(51) Int. Cl.: A61K 31/525, A61K 31/195, A61K 31/12, A61K 31/205, A61K 31/455, A23L 1/20

(54) **DIETARY SUPPLEMENT ENERGY-PROVIDING TO SKELETAL MUSCLES AND PROTECTING THE CARDIOVASCULAR TRACT**
ENERGIELIEFERNDE NAHRUNGSERGÄNZUNG FÜR SKELETTMUSKULATUR UND ZUM SCHUTZ DES HERZ-KREISLAUF-SYSTEMS
SUPPLEMENT ALIMENTAIRE ALIMENTANT LES MUSCLES SQUELETTIQUE EN ENERGIE ET PROTEGE LE TRACTUS CARDIOVASCULAIRE

(30) Priority: 04.04.2000 IT RM000165
(43) Date of publication of application: 29.05.2002
(73) Proprietor: SIGMA-TAU Industrie Farmaceutiche Riunite S.p.A., 00144 Roma (IT)
(72) Inventor: GAETANI, Franco, I-00040 Ariccia (IT)
(74) Representative: Cavattoni, Fabio
(86) International application number: PCT/IT2001/000167
(87) International publication number: WO 2001/074361

(56) References cited:
- US-A- 3 219 454
- US-A- 3 689 641
- US-A- 4 237 118
- US-A- 5 889 062

## Description

The present invention relates to an energy-giving dietary supplement aimed particularly at facilitating the adaptation of skeletal and cardiac muscle of subjects engaging in physical and/or recreational activity that may be particularly intense and prolonged.

Anyone engaging in sports activities, whether professionally or as an amateur, wishes to achieve as soon as possible and maintain for as long as possible the maximum degree of adaptation of the skeletal muscles to the ability to sustain prolonged periods of intense physical activity.

The quest for optimal physical fitness may favour the inappropriate use of drugs, particularly steroids. It is well known that such drugs may enhance protein synthesis and consequently boost the growth of muscle masses to a greater extent than can be achieved by training and dieting. The use of such drugs, however, is unquestionably damaging to health as well as being illegal when practised in professional sport.

It is, therefore, obvious that the only correct way to achieve the above-mentioned goal consists in engaging in lengthy training schedules backed up by suitable, properly supplemented diets.

Thus, more or less recently, various dietary supplements have been proposed aimed at reinforcing the diets of individuals engaging in intense physical activity whether at the professional or amateur level. The vast majority of these supplements devote particular attention to the metabolism of the skeletal muscle which requires a vast range of nutrients for protein synthesis, mainly including amino acids. In fact, since almost all amino acids, whether essential or non-essential, are substrates needed by the muscle cells for such synthesis, dietary supplements have been marketed now for some time containing mixtures of amino acids in various weight-to-weight ratios in combination with other active ingredients and nutrients (see, for example, US Patents 4,687,782 and 5,292,538).

With other dietary supplements, on the other hand, the attention is focused rather on the production of energy and thus of ATP. The ingredients characterizing these supplements are therefore mainly coenzyme Q₁₀ and creatine.

Coenzyme Q₁₀ plays a fundamental role in the transport of electrons along the mitochondrial respiratory chain, which is necessary for the energy transformations needed for ATP production.

The physiological function of creatine, which is partly biosynthesized in the liver and kidneys and partly ingested with the diet, is also extremely important in energy terms: in muscle, but also in the brain, liver and kidneys, creatine reversibly takes up the phosphoric group of ATP and plays a role as a reserve of phosphoric radicals rich in energy. The importance of this reaction stems from the fact that ATP cannot accumulate in tissues above a very modest limit. It is the phosphocreatine present in tissues in amounts roughly five-fold higher than ATP, that ensures its supply. In fact, after even only moderate physical exercise, phosphocreatine diminishes in skeletal muscle to a much more marked extent than ATP, demonstrating that phosphocreatine rephosphorylates ATP, as the ATP is dephosphorylated. When the rate of metabolic production of ATP exceeds its rate of use, phosphocreatine is formed. Phosphocreatine thus constitutes a store of immediately utilizable energy suitable for "buffering" energy needs above the ATP synthesis rate in phosphorylative metabolic processes.

In brief, with the existing dietary supplements there is a tendency, on the one hand, to enhance muscle mass and, on the other, to constitute energy reserves that make available immediately "consumable" energy when the intensity of the physical effort requires it.

The muscle enhancement and the increased availability of energy favoured by these known food supplements may, however, cause even severe side effects, particularly in subjects who, since they do not practise sport professionally and thus are not subjected periodically to thorough check-ups, may be induced to engage in physical performances exceeding their physiological resistance limits without them necessarily perceiving this situation.

Such subjects constitute the majority of users of dietary supplements and a considerable proportion of them are made up of individuals who are no longer young or may be decidedly elderly, who very rarely undergo medical check-ups to ascertain their suitability for the physical activity they undertake and to establish the limits of intensity and effort beyond which it is dangerous to push oneself.

Since it is particularly the cardiovascular system that is most strongly stressed by any type of physical or sporting activity, there can be little doubt as to the obvious danger to which these users are exposing themselves, in that their propensity to sustain loads of fatigue and physical stress disproportionate to the state and integrity of the cardiovascular apparatus may be increased considerably by consuming such energy-giving supplements.

There is, therefore, a perceived need for a dietary supplement which, on the one hand, has an energy-giving and strengthening effect on skeletal muscle and, on the other, exerts at the same time a protective, tonic effect on the user's cardiovascular apparatus.

The aim of the present invention is to provide just such a dietary supplement.

One object of the present invention is, therefore, a dietary supplement endowed with a potent strengthening and energy-giving effect on skeletal muscle and, at the same time, a protective, tonic effect on the cardiovascular apparatus of individuals engaging in sporting and/or recreational activities that may require intense, prolonged physical effort, the characterizing components of which, in combination or packaged separately, comprise:
a) propionyl L-carnitine or one of its pharmacologically acceptable salts;
b) coenzyme Q₁₀;
c) nicotinamide;
d) riboflavin, and
e) pantothenic acid.

The weight-to-weight ratio of components (a):(b):(c):(d):(e) ranges from 10:0.04:0.08:0.08:0.4 to 1:4:10:4:20 and preferably from 10:2:5:2:2 to 1:1:4:1:5.

The activity of the "carnitines" in general, and of propionyl L-carnitine in particular, on lipid metabolism is well known, as is their anti-atherosclerotic action and their action on lipid metabolism disorders. Propionyl L-carnitine, however, differs from the other "carnitines" in its specific cardiovascular activity, despite participating, like the other "carnitines", above all at mitochondrial level, in the important metabolic role related to the β-oxidation of fatty acids and ATP synthesis.

Propionyl L-carnitine takes part in all the metabolic activities characteristic of the "carnitines", but, unlike the others, presents a more pronounced activity at the vascular level, and particularly at the level of the peripheral circulation, thus presenting itself as a valid therapeutic agent for the prevention and treatment of various peripheral vasculopathies. Propionyl L-carnitine is also superior to the other carnitines in conditions in which the other carnitines are unable to act, and this particular feature is related to its more direct metabolic intervention in the processes of energy utilization at the mitochondrial level and to the presence of the propionyl group which distinguishes its pharmacological effect from that of other similar molecules to such an extent as to make it a chemical entity in its own right, with superior and different properties to those of the other carnitines.

Propionyl L-carnitine is a naturally occurring component of the pool of carnitines and is synthesized by means of carnitine acetyl-transferase starting from propionyl-Coenzyme A.

Its administration to human subjects leads to an increase in plasma concentrations of propionyl L-carnitine which in turn causes an increase in plasma concentrations of L-carnitine which regulate its content in the cells with an increase in their oxidative effect on fatty acids and utilization of glucose. In addition, muscular carnitine transferase possesses a greater affinity for propionyl L-carnitine than for L-carnitine, and consequently propionyl L-carnitine possesses a higher degree of specificity for cardiac and skeletal muscle. Transporting the propionyl group, propionyl L-carnitine increases the uptake of this component by the muscle cells, particularly those of the myocardium. This may be of particular importance, since propionate can be used by the mitochondria as an anaplerotic substrate and supply energy in anaerobic conditions. It should be recalled that propionate cannot be used alone on account of its side effects.

Apart from these metabolic effects, it should also be recalled that, owing to its alkanoyl chain, propionyl L-carnitine exerts a specific pharmacological action by activating peripheral vasodilatation and myocardial inotropism in conditions in which the other carnitines are inactive.

In addition to propionyl L-carnitine, the dietary supplement can further comprise a "carnitine" selected from the group consisting of L-carnitine, acetyl L-carnitine, valeryl L-carnitine, isovaleryl L-carnitine and butyryl L-carnitine or their pharmacologically acceptable salts.

What is meant by a pharmacologically acceptable salt of L-carnitine or of an alkanoyl L-carnitine is any salt of these with an acid which does not give rise to unwanted toxic or side effects. These acids are well known to pharmacologists and to experts in pharmaceutical technology.

Examples of such salts, but by no means exclusively these, are the following: chloride; bromide; iodide; aspartate, acid aspartate; citrate, acid citrate; tartrate; phosphate, acid phosphate; fumarate, acid fumarate; glycerophosphate; glucose phosphate; lactate; maleate, acid maleate; mucate; orotate; oxalate, acid oxalate; sulphate, acid sulphate; trichloroacetate; trifluoroacetate and methane sulphonate.

A list of FDA-approved pharmacologically acceptable acids is given in Int. J. Pharm., 33, 1986, 201-217, the latter publication being incorporated in the present description for reference purposes.

For the preparation of solid administration forms, such as, for example, tablets, pills, capsules and granulates, the use of non-hygroscopic salts is preferred. The preferred non-hygroscopic salts of propionyl L-carnitine and of any other alkanoyl L-carnitines present are the mucates (or galactarates), disclosed in US Patent 5,952,379.

Whenever, in the above-mentioned solid administration forms, L-carnitine is also present, the preferred salt of this carnitine is the acid fumarate described in US Patent 4,602,039. US Patent 5,308,832 discloses compositions with pantothenic acid, riboflavine and carnitine.

In addition to its characteristics of stability and lack of hygroscopicity, L-carnitine fumarate exerts a double protective action with regard to protein metabolism: through a direct increase in intermediate metabolism, it indirectly stimulates protein biosynthesis and, as a result of the mobilization of fatty acids, induces a sparing/protective effect on the muscle protein components.

The dietary supplement according to the invention may further comprise one or more of the following components:
f) an amino acid selected from the group consisting of valine, leucine and isoleucine or mixtures thereof;
g) a creatine selected from the group consisting of creatine and phosphocreatine or mixtures thereof.

The dietary supplement of the present invention in unit dose form contains:

| | | |
|---|---|---|
| propionyl L-carnitine | from 50 mg | to 2,000 mg |
| coenzyme Q₁₀ | from 5 mg | to 200 mg |
| nicotinamide | from 10 mg | to 500 mg |
| riboflavin | from 5 mg | to 200 mg |
| pantothenic acid | from 10 mg | to 1,000 mg |

For example, a formulation suitable for tablets is the following:

| | |
|---|---|
| propionyl L-carnitine · HCl | 250 mg |
| coenzyme Qio | 20 mg |
| nicotinamide | 50 mg |
| riboflavin | 20 mg |
| pantothenic acid | 20 mg |

The supplement may further comprise mineral salts, such as, for example, disodium citrate, monopotassium phosphate, calcium lactate and magnesium taurate. The dietary supplement of the present invention is suitable for oral administration. The supplement, even when comprising the above-mentioned amino acids, must not be used as a single or main source of nutrition on a day-to-day basis.

The complementary part of the diet will, therefore, consist of appropriate amino acids, carbohydrates, lipids, vitamins and minerals.

The amount of dietary supplement taken daily may vary within broad limits, depending, for example, on the subject's age and weight, or upon the intensity and complexity of the training schedule or the physical activity the individual engages in.

The potent energy-giving effect on skeletal muscle and, at the same time, the protective effect on the cardiovascular system that is achieved with the dietary of the present invention has been shown by several pharmacological tests (some of which are described here below) selected in such a way as to be strongly predictive for the practical use of the supplement in the human field. In these tests, the animals treated with a composition according to the invention were administered the tablet formulation previously described at the dose of 50 mg/kg/day for seven weeks.

### Determination of horizontal locomotor activity

Motor activity is a good indicator for studying the effects of pharmacological agents. The motor performance of the skeletal muscles of treated animals and control animals was assessed at the end of treatment. Motor activity was measured by placing the animals individually in motility cages (Omnitech Digisean Animal Activity Monitor, Columbus OH, USA). Each cage had positioned in the righthand corner 2 sets of 16 photocells projecting horizontal infrared rays at a distance of 2.5 cm from one another and placed 2 cm above the bottom of the cage. Motor activity was determined in terms of mean speed as the relationship between horizontal activity (distance travelled) and time taken. All counts were totalized and recorded automatically for a period of 20 minutes.

### Results

### Locomotor speed of rats treated with vehicle (control animals) and rats treated with the composition according to the invention

At the end of treatment, locomotor activity was measured in male and female control rats and in male and female rats treated with the composition according to the invention. The male rats that had received the composition according to the invention showed a speed in horizontal movements equal to 2.86 ± 0.09 cm × s⁻¹ × 100 g body weight⁻¹ (n=10) with a 19% increase compared to those treated with vehicle (2.40 ± 0.07 cm × s⁻¹ × 100 g body weight, n=10).

This effect was more marked in the female animals. In fact, female rats treated with the composition according to the invention showed a speed of 5.4 ± 0.22 cm × s⁻¹ × 100 g body weight (n=5), which was higher (25%) (P<0.05) than that recorded in rats treated with the vehicle (3.66 ± 0.16 cm × s⁻¹ × 100 g body weight, n=5).

### Experimental studies on papillary muscle

### Methods

### General procedures

In the course of deep anaesthesia with ether, the chest was opened and the heart was rapidly excised and placed in a modified Krebs solution pre-equilibrated with 95% O₂ and 5% CO₂, at ambient temperature. The composition of the Krebs solution was the following (mM): NaCl 123, NaHCO₃ 20, MgSO₄ 0.8, KCl 6, CaCl₂ 2.52, KH₂PO₄ 1.16, glucose 11.98. The heart was massaged to induce the heart beat and expel any residues of blood from the ventricles. The left papillary muscle was excised with part of the ventricle and transferred within 2 minutes to a muscle container equipped with a central perfusion channel measuring 4 mm in diameter through which the Krebs solution was perfused at a rate of 2.5 ml/min and bubbled vigorously by insufflating 95% O₂ and 5% CO₂. (Fig. 3). The oxygen was supplied via a thin gas dispersion tube immersed in the muscle container. A plastic septum was interposed between the papillary muscle and the supply of oxygen. This arrangement, similar to that described by Blinks ("Convenient apparatus for recording contractions of isolated heart muscle", J. Appl. Physiol. 4: 755-757, 1965) allows good oxygenation and stirring without any mechanical perturbation. The muscle container was kept at a temperature of 32°C by means of the use of a water circulation pump (Basile, Comerio, Italy, mod. 4050).

### Determination of length/tension

The papillary muscle was held at its non-tendinous end by a clamp connected up to a part of the ventricular wall excised with the muscle, as suggested by Molé ("Increased contractile potential of papillary muscles from exercise-trained rat hearts", Am. Physiol. Soc., 234(4): 421-425, 1978). This was to avoid damaging the papillary muscle. The muscle clamp was firmly fixed to bottom of the bath. The upper tendinous end of the muscle was connected up by means of a thin gold chain to a force transducer (Mod. WPI Fort 10, 2200 µV/V/g; ADInstruments Pty Ltd, Australia) for the measurement of isometric tension. The transducer was mounted on a mobile support which allowed minimal 5 µm increases in length. The muscle was stimulated at a frequency of 3 pulses per min⁻¹ with rectangular or 5 msec two-phase pulses by means of a pair of platinum electrodes which were set up with the preparation; the distance between the electrodes was 7 mm. The electrodes were fed with a transverse electrical stimulation field by means of high-output constant current (Multiplexing Pulse Amplifier, Basile - Italy). A Lab-type output stimulator (ADInstrument, Australia) controlled the duration and frequency of the electrical stimuli. The stimulus intensity barely exceeded the perceptibility threshold, corresponding to a current density of 80-120 mA. For the stabilization, the papillary muscle was contracted under a zero load with a stimulus frequency of 3 pulses per min⁻¹. After stabilization, the transducer support to which the muscle was connected was adjusted until a tension of 3 mN (pre-load) was recorded. At this point, the length of the papillary muscle was measured by means of a stereomicroscope (Wild M 3B, Heerbrug, Switzerland, 40x) equipped with an ocular micrometer. This length was defined as the "initial length" (Lᵣ). The basal value recorded was considered as representing a tension of 0 mN.

To obtain length/tension responses, the papillary muscle was stretched with increments of 0.05 Lᵣ from 1.00 Lᵣ to 1.30 Lᵣ. The muscle was given a chance to restore equilibrium conditions for five minutes after each increment in length, so as to allow yielding under load. At 1.30 Lᵣ the process was inverted and the length of the muscle was decreased at 5 minute intervals, with decrements comparable to the previous increment. For each length, the transducer recorded both the resting force (passive) exerted by the muscle as the result of stretching and the force developed (active) in response to electrical stimulation.

### Determination of force/speed

The force/speed determination was done by means of the classic isotonic post-load technique. The shortening of the muscle was measured by means of a Basil 7006 linear shift transducer (moment of inertia 35 g per cm², minimum effective force < 0.1 g). The lever arm of the transducer (distance between the fulcrum of the lever and the attachment of the organ examined: 10 cm, operating range ± 15°) consisted of a thin wall of conical tubing made of carbon fibre. The loading of the lever arm was done by means of a tungsten alloy cylindrical counterweight which moved along a scale, thus providing for load variations of 0.01 g/step. Repeated stimulations were transmitted to the papillary muscle. The shortening was recorded at each shock stimulus; the load was increased by degrees with a constant increment of 0.05 g. The procedure continued until the shift could not be reliably distinguished from noise.

The signals of the isometric and isotonic transducers were recorded and analyzed by a computer (Pentium Pro 32 MB ram) equipped with an analog-to-digital conversion program (Mac. Lab. ADInstruments, Australia) which ran with a V.3.0 software chart.

### Determination of muscle shortening, work and power

On the basis of the isotonic experiments the shortening work, the shortening power and the shortened tract were calculated. The work was calculated as the product of the shortening and the load lifted. The power was calculated as the product of the speed and the load lifted.

### Results

### Length/tension relationships

Treatment with the composition according to the invention resulted in a significant positive inotropic effect, as indicated by the increase in developed or active tension up to the levels observed in the papillary muscles of control rats. In fact, the active tension was greater for the treated muscles over the entire range of muscle lengths studied.

At the final length (1.30 Lᵣ) the maximum active tension value of 13.42 ± 0.88 mN × mg dry tissue weight⁻¹ for the papillary muscle of the left ventricle was significantly greater (P< 0.05) than the value in control animals of 10.64 ± 1.32 mN × mg dry tissue weight⁻¹.

Treatment with the composition according to the invention did not significantly modify the passive length-tension of papillary muscle.

### Response to stimulation frequencies

The positive inotropic effect of the composition according to the invention was also observed in response to various electrical stimulation values.

The papillary muscle treated with the composition according to the invention showed a marked increase in developed tension over the entire range of frequencies tested.

### Force-speed relationships

Force-speed relationships were determined in left ventricular papillary muscle of both control animals and animals treated with the composition according to the invention.

The speed was normalized by dividing the muscle shortening per second by L_{T}.

A positive inotropic effect as a result of treatment with the composition according to the invention was, moreover, observed on the shortening rate, on the shortening and on the work and power curves. Force-speed curves were obtained as a function of increasing post-load from normal rats and from rats treated with the composition according to the invention. Inverse relationships between force and speed were shown in both groups of left ventricular papillary muscle. The shortening rate was greater in the muscle of animals treated with the composition according to the invention (n=5) than in the control animals (n=5) at post-loads ranging from 0.5 to 1.25 mN (P<0.05).

At the lowest post-load (0.5 mN) the muscle shortening in normal control rats was 0.08 ± 0.029 mm × muscle length⁻¹, whereas in those treated with the composition according to the invention the shortening was 2.5 times greater (0.2 ± 0.06 mm × muscle length⁻¹).

### Work and power

The work done by the papillary muscle at various loads was determined. The maximum physical work (0.11 ± 0.039 µJ × muscle length⁻¹) was studied in animals treated with the composition according to the invention placed under a load of approximately 25% of the maximum load that the muscles could lift (Po) (3mN), whereas in the control animals the maximum work (0.054 ± µJ × muscle length⁻¹) was obtained at 38% of the Po (3.23 mN). In papillary muscle treated with the composition according to the invention, the increased work curve differed significantly (P<00.1) as compared to the controls. The maximum amount of work recorded in the animals treated with the composition according to the invention was approximately two-fold greater than that obtained in the control animals.

As regards the muscle power, the maximum value (0.91 ± 0.29 µW × muscle length⁻¹) was 1.5-fold higher in the animals treated with the composition according to the invention than in the control animals (0.59 ± 0.24 µW × muscle length⁻¹).

## Claims

1. A dietary supplement comprising as active ingredients a combination of admixed or separately packaged:
a) propionyl L-carnitine or a pharmacologically acceptable salt thereof;
b) coenzyme Q₁₀;
c) nicotinamide;
d) riboflavin; and
e) pantothenic acid.

2. The dietary supplement of claim 1, wherein component (a) further comprises a "carnitine" selected from the group consisting of L-carnitine, acetyl-L-carnitine, valeryl-L-carnitine, isovaleryl-L-carnitine and butyryl-L-carnitine or the pharmacologically acceptable salts thereof or mixtures thereof.

3. The dietary supplement of claims 1 or 2 wherein the pharmacologically acceptable salt is selected from the group consisting of: chloride; bromide; iodide; aspartate, acid aspartate; citrate, acid citrate; phosphate, acid phosphate; fumarate, acid fumarate; glycerophosphate; glucose phosphate; lactate; maleate, acid maleate; mucate; orotate; oxalate; acid oxalate; sulphate, acid sulphate; tartrate; trichloroacetate; trifluoroacetate and methane sulphonate.

4. The dietary supplement of claims 1, 2 or 3, which further comprises at least one of the following components:
f) an aminoacid selected from the group consisting of valine, leucine, isoleucine or mixtures thereof;
g) a creatine selected from the group consisting of creatine and phosphocreatine or mixtures thereof.

5. The dietary supplement of claim 1 wherein the weight ratio (a):(b):(c):(d):(e) ranges from 10:0.04:0.08:0.08:0.4 to 1:4:10:4:20.

6. The dietary supplement of claim 5 wherein the weight ratio (a):(b):(c):(d):(e) ranges from 10:2:5:2:2 to 1:1:4:1:5.

7. The dietary supplement of anyone of the preceding claims in the form of tablets, lozenges, pills, capsules and granulates.

8. The dietary supplement of claim 7 in unit dosage form comprising:
| | | |
|---|---|---|
| propionyl L-carnitine | from 50 mg | to 2,000 mg |
| coenzyme Q₁₀ | from 5 mg | to 200 mg |
| nicotinamide | from 10 mg | to 500 mg |
| riboflavin | from 5 mg | to 200 mg |
| pantothenic acid | from 10 mg | to 1,000 mg. |

## Patentansprüche

1. Nahrungsergänzungsmittel, umfassend als aktive Ingredienzien eine Kombination von zugemischtem oder getrennt verpacktem/zugemischter oder getrennt verpackter:
a) Propionyl-L-carnitin oder einem pharmakologisch akzeptablen Salz davon;
b) Cöenzym Q₁₀;
c) Nicotinamid
d) Riboflavin; und
e) Pantothensäure.

2. Nahrungsergänzungsmittel nach Anspruch 1, wobei-Komponente (a) außerdem ein "Carnitin", das aus der Gruppe bestehend aus L-Carnitin, Acetyl-L-carnitin, Valeryl-L-carnitin, Isovaleryl-L-carnitin und Butyryl-L-carnitin ausgewählt ist, oder die pharmakologisch akzeptablen Salze davon oder Gemische davon umfasst.

3. Nahrungsergänzungsmittel nach Anspruch 1 oder 2, wobei das pharmakologisch akzeptable Salz aus der Gruppe bestehend aus Chlorid; Bromid; Iodid; Aspartat, saurem Aspartat; Citrat, saurem Citrat; Phosphat, saurem Phosphat; Fumarat, saurem Fumarat; Glycerophosphat; Glucosephosphat; Lactat; Maleat, saurem Maleat; Mucat; Orotat; Oxalat, saurem Oxalat; Sulfat, saurem Sulfat; Tartrat; Trichloracetat; Trifluoracetat und Methansulfonat ausgewählt ist.

4. Nahrungsergänzungsmittel nach Anspruch 1, 2 oder 3, das außerdem wenigstens eine der folgende Komponenten umfasst:
f) eine Aminosäure, ausgewählt aus der Gruppe bestehend aus Valin, Leucin, Isoleucin oder Gemischen davon;
g) ein Creatin, ausgewählt aus der Gruppe bestehend aus Creatin und Phosphocreatin oder Gemischen davon.

5. Nahrungsergänzungsmittel nach Anspruch 1, wobei das Gewichtsverhältnis (a) : (b) : (c) : (d) : (e) im Bereich von 10:0,04:0,08:0,08:0,4 bis 1:4:10:4:20 liegt.

6. Nahrungsergänzungsmittel nach Anspruch 5, wobei das Gewichtsverhältnis (a) : (b) : (c) : (d) : (e) im Bereich von 10:2:5:2:2 bis 1:1:4:1:5 liegt.

7. Nahrungsergänzungsmittel nach einem der vorangehenden Ansprüche in Form von Tabletten, Lutschbonbons, Pillen, Kapseln und Granulaten.

8. Nahrungsergänzungsmittel nach Anspruch 7 in Einheitsdosierungsform, umfassend:
| | | |
|---|---|---|
| Propionyl-L-carnitin | von 50 mg | bis 2.000 mg |
| Coenzym Q₁₀ | von 5 mg | bis 200 mg |
| Nicotinamid | von 10 mg | bis 500 mg |
| Riboflavin | von 5 mg | bis 200 mg |
| Pantothensäure | von 10 mg | bis 1.000 mg. |

## Revendications

1. Complément alimentaire comprenant, en tant qu'ingrédients actifs, une combinaison, sous la forme d'un mélange ou d'un conditionnement séparé :
(a) de propionyl-L-carnitine ou d'un sel pharmacologiquement acceptable de celle-ci ;
(b) de coenzyme Q₁₀ ;
(c) de nicotinamide ;
(d) de riboflavine; et
(e) d'acide pantothénique.

2. Complément alimentaire selon la revendication 1, dans lequel le composé (a) comprend en outre une "carnitine" choisie parmi le groupe comprenant la L-carnitine, l'acétyl-L-carnitine, la valéryl-L-carnitine, l'isovalésyl-L-carnitine et la butyryl-L-carnitine, ou leurs sels pharmacologiquement acceptables ou des mélanges de ceux-ci.

3. Complément alimentaire selon la revendication 1 ou 2, dans lequel le sel pharmacologiquement acceptable est choisi parmi le groupe comprenant : le chlorure ; le bromure; l'iodure ; l'aspartate, l'aspartate acide ; le citrate, le citrate acide; le tartrate; le phosphate, le phosphate acide; le fumarate, le fumarate acide ; le glycérophosphate ; le phosphate de glucose ; le lactate ; le maléate, le maléate acide ; le mucate ; l'orotate ; l'oxalate, l'oxalate acide ; le sulfate, le sulfate acide ; le tartrate ; le trichloroacétate ; le trifluoroacétate et le sulfonate de méthane.

4. Complément alimentaire selon les revendications 1, 2 ou 3, qui comprend en outre au moins un des composés suivants :
(f) un acide aminé choisi dans le groupe constitué de la valine, de la leucine, de l'isoleucine ou de mélanges de ceux-ci ; et
(e) une créatine choisie dans le groupe constitué de la créatine et de la phosphocréatine ou de mélanges de ceux-ci.

5. Complément alimentaire selon la revendication 1, dans lequel le rapport massique (a):(b):(c):(d):(e) varie de 10:0,04:0,08:0,08:0,4 à 1:4:10:4:20.

6. Complément alimentaire selon la revendication 5, dans lequel le rapport massique (a):(b):(c):(d):(e) varie de 10:2:5:2:2 à 1:1:4:1:5.

7. Complément alimentaire selon l'une quelconque des revendications précédentes, sous la forme de comprimés, de tablettes, de pilules, de gélules ou de granules.

8. Complément alimentaire selon la revendication 7, sous forme de dose unitaire, comprenant :
| | | |
|---|---|---|
| de la propionyl-L-carnitkne | à raison de 50 mg | à 2 000 mg |
| de la coenzyme Q₁₀ | à raison de 5 mg | à 200 mg |
| du nicotinamide | à raison de 10 mg | à 500 mg |
| de la riboflavine | à raison de 5 mg | à 200 mg |
| de l'acide pautothénique | à raison de 10 mg | à 1 000 mg |
